# EUROPEAN PATENT SPECIFICATION

(11) **EP 3 858 410 B1**
(45) Date of publication and mention of the grant of the patent: **26.10.2022**
(21) Application number: 21151288.4
(22) Date of filing: 13.01.2021
(51) Int. Cl.: A61M 16/00, A61M 16/06

(54) **ARTIFICIAL VENTILATION SYSTEM FOR PATIENTS**
KÜNSTLICHES BEATMUNGSSYSTEM FÜR PATIENTEN
SYSTÈME DE RESPIRATION ARTIFICIELLE POUR PATIENTS

(30) Priority: 30.01.2020 IT 202000001804
(43) Date of publication of application: 04.08.2021
(73) Proprietor: Intersurgical S.P.A., 41037 Mirandola, Modena (IT)
(72) Inventor: NAVALESI, Paolo, 20149 MILANO (IT); LONGHINI, Federico, 28021 BORGOMANERO (NO) (IT); ROSSI, Paolo, 41037 MIRANDOLA (MO) (IT)
(74) Representative: Corradini, Corrado

(56) References cited:
- EP-A1- 3 320 941
- WO-A1-2011/078703
- WO-A1-2016/157105
- WO-A1-2016/159787
- US-A1- 2012 125 338

## Description

### TECHNICAL FIELD

The present invention relates to an artificial ventilation system for patients.

In particular, the invention relates to a continuous positive airway pressure (CPAP) ventilation system comprising a nasal cannula for oxygen therapy.

### PRIOR ART

As is known, for artificial ventilation such as non-invasive ventilation (NIV) or continuous positive airway pressure (CPAP) mechanical ventilation, of patients in general, systems have been used comprising various types of patient interfaces, such as masks or helmets which, in general, are adapted to be coupled sealingly with the face or neck of a patient.

Such systems for artificial respiration are used in order to allow patients to breathe who would not otherwise be able to breathe independently.

For that purpose, through the patient interface, the patients are administered a mixture of breathable gases, e.g. air or oxygen-enriched air or pure oxygen, having a positive pressure that pushes such mixture into the lungs.

Furthermore, the use of nasal cannulae provided with nozzles for the direct administration of a breathable gas into the patient's nose is known, which can be used alone or combined with a high-pressure therapy administration system.

Nasal cannulae are used, for example, for the application of humidified or high flow oxygen therapy which consists of the administration of a flow of breathable gas greater than that required by the patient. Thus the patient is supplied with a FiO₂ (fraction of inspired oxygen) in a high percentage. An example of such known systems is disclosed in prior art document nr. EP3320941A1.

Artificial respiration systems comprising a mask and a nasal cannula arranged inside the volume of the mask are disclosed in WO 2011/078703, WO 2016/157105 and US 2012/125338.

A strongly felt need in the sector is that of allowing the effective, cheap, safe and functional connection between a patient interface, generally suitable for therapy for patients who require a high pressure breathable gas, and a nasal cannula, i.e. a nasal cannula for the application of humidified or high flow oxygen therapy so as to guarantee the success of combined therapy.

An object of the present invention is that of fulfilling this need, within the scope of a simple, rational and affordable solution that is safe for the patient.

In particular, an object of the present invention is that of making available an artificial ventilation system for patients that facilitates, technically and in terms of speed, for the appointed personnel and/or for the patient, the connection of said nasal cannula to the patient interface and that, at the same time, guarantees a pneumatic seal of the patient interface that is adaptable to all types of patient interfaces and/or nasal cannulae (for the application of humidified or high flow oxygen therapy) existing on the market.

Such object is achieved by the features of the invention indicated in the independent claim. The dependent claims outline preferred and/or particularly advantageous aspects of the invention.

### DISCLOSURE OF THE INVENTION

The invention particularly makes available an artificial ventilation system, comprising:
- a patient interface adapted to contain a volume in communication with at least the mouth and nose of a patient, wherein the patient interface is provided with a through hole;
- a nasal cannula connected to the through hole of the patient interface; and
- a connection element snugly fitted into the through hole and sealingly fitted onto the nasal cannula, wherein the connection element is elastically deformable.

Thanks to this solution, a system is made available that combines humidified oxygen therapy with CPAP therapy in a particularly effective, simple and cost contained way.

In particular, the connection element with which said system is provided can interconnect easily, intuitively, safely and quickly the patient interface and the nasal cannula, for the efficient and convenient use of said combined therapy, conveniently and easily for the patient and/or the appointed personnel, at the same time guaranteeing the pneumatic seal of the system itself.

Furthermore, the nasal cannula enables effective "flushing" of the airways, reducing the re-breathing of CO₂ within the volume enclosed by the patient interface.

According to a first embodiment of the invention, the patient interface can comprise a helmet provided with a container body in which the (entire) head of the patient can be housed and a collar that can be sealingly coupled to the patient's neck.

In this way, a patient interface is made available, provided with high therapeutic efficacy and that is well tolerated by patients, also for prolonged periods of administration of the therapy(ies); furthermore, the breathing volume for the patient is maximized with the consequent possibility to maximize the oxygenation yield of the patient and minimize the re-breathing of CO₂.

According to a second embodiment of the invention, the patient interface can comprise a mask, preferably a full-face mask provided with a perimeter edge that can be coupled to the patient's face and configured to inscribe the patient's mouth, nose and eyes.

It is not excluded that the mask may be a mask of the oronasal type, wherein the perimeter edge can be sealingly coupled to the patient's face and is configured to inscribe (only) the patient's mouth and nose, leaving the eyes uncovered.

Thanks to such solution, a patient interface is made available, characterized by reduced dimensions, ease and speed of use (also in emergency situations) and functional to the administration of therapy(ies). The invention envisages that the connection element is elastically deformable.

In this way, a connection element is made available that adapts to measure to all types of patient interfaces and/or nasal cannulae (for the application of humidified or high flow oxygen therapy) existing on the market, guaranteeing at the same time effective sealing coupling between the nasal cannula and the patient interface.

A further aspect of the invention envisages that the connection element can comprise a (cylindrical) sleeve that can be snugly fitted into the through hole and provided with a central cavity (preferably, but not necessarily, cylindrical and coaxial to the cylindrical sleeve and, therefore, to the through hole).

Thanks to such solution, the connection element, through the sleeve with which it is provided, can be easily inserted into the through hole and, therefore, connected to the patient interface so as to guarantee the pneumatic seal between the connection element and the patient interface; furthermore, as it has a central cavity itself, it can in turn be fitted onto the nasal cannula allowing the pneumatic seal to be guaranteed between the connection element and the nasal cannula itself.

Furthermore, an aspect of the invention envisages that the sleeve can comprise two flaps that surround and define the central cavity, wherein the two flaps are operatively switchable between a closing configuration, in which the central cavity has a minimum (cross) section, and an opening configuration, in which the central cavity has a larger (cross) section than the minimum (cross) section.

In this way, the sleeve can be easily fitted radially onto the nasal cannula, without being obliged to axially insert the central cavity thereof with an axial end of the nasal cannula. Furthermore, thanks to the "openable" configuration of the sleeve it is possible to have ample freedom of installation/connection between the nasal cannula and the patient interface making such operation particularly convenient for the appointed personnel.

For example, the nasal cannula can be installed in advance in proximity to the patient's nose, initially leaving the nasal cannula itself free from the connection with the patient interface and, only subsequently can the nasal cannula be connected to the patient interface, i.e. to the through hole thereof, by means of the interposition of the sleeve. Furthermore, thanks to such solution, the installation of the system on the patient is made particularly easy for the appointed personnel and/or for the patient, both technically and in terms of connection speed and yield.

According to a further aspect of the invention, the two flaps can be made of a single body and can each have a proximal end that derives from the proximal end of the other flap and an opposing distal end separated from the distal end of the other flap.

Thanks to such solution, the sleeve is particularly convenient for the personnel and easy to produce for the manufacturer.

An aspect of the invention further envisages that the patient interface can comprise a through opening, adapted to place in communication the outside of the helmet with the volume enclosed thereby, wherein the through opening is sized such as to be able to be crossed by a hand and closed sealingly by a plug.

In this way, the appointed personnel has sufficient available space to manoeuvre on the patient, also while the patient is wearing the mask, i.e. straight before, during or straight after the administration of the therapy(ies).

Furthermore, the plug that sealingly closes said through opening can be provided with said through hole, to which the nasal cannula can be connected and inside which the aforesaid connection element (the sleeve) can be snugly fitted.

In this way, it is possible to enable the connection of the nasal cannula to the patient interface in a convenient and detachable position from the patient interface in order to meet the most varied needs of the appointed personnel.

A further aspect of the invention envisages that the patient interface can comprise a means for the introduction of a breathable gas into the volume enclosed by the patient interface itself.

For example, the introduction means may be separate from the nasal cannula or coincide with the nasal cannula.

Furthermore, an aspect of the invention envisages that the patient interface can comprise a means for the outflow of the gases exhaled by the patient towards the outside of the volume contained by the patient interface.

In this way, it is possible to supply a breathable gas to the patient that occupies the volume contained by the patient interface, allowing at the same time the outflow of the gases exhaled by the patient towards the outside of the volume itself.

According to a further aspect of the invention, the nasal cannula can be provided with a hose to a first end of which a nasal dispenser is fixed for the administration of a breathable gas directly into the patient's nose.

Thanks to such a solution, the nasal cannula is conformed so as to enable the appropriate use of the therapy for which it is intended.

According to a further aspect of the invention, at least the nasal dispenser of the nasal cannula and, preferably but not necessarily, at least a portion of the hose are arranged within the volume contained by the patient interface.

In this way, the nasal cannula is connected to the patient interface in a stable and functional way to the use of said therapy.

Furthermore, a further aspect of the invention envisages that the hose of the nasal cannula can be at least partially breathable for a water vapour contained in the breathable gas.

Thanks to such a solution, the water vapour transported by the nasal cannula, together with the oxygen, is also partially released, through the hose, into the volume contained by the patient interface, allowing synergy between the two respiratory therapies that can be administered through the artificial ventilation system.

Advantageously, a second end of the nasal cannula, opposite the first end provided with the nasal dispenser, can be provided with a (standard) connector for the connection to a (dedicated or common) supply circuit of a breathable gas, e.g. a circuit for high flow oxygen therapy, which connector can be arranged or engageable from the outside of the patient interface.

### BRIEF DESCRIPTION OF THE DRAWINGS

Further features and advantages of the invention will be more apparent after reading the following description provided by way of a non-limiting example, with the aid of the figures shown in the accompanying drawings.
Figure 1 is an axonometric view of the artificial ventilation system according to a first configuration of a first embodiment of the invention.
Figure 2 is an axonometric view of the artificial ventilation system according to a second configuration of the first embodiment of the invention.
Figure 3 is an axonometric view of the artificial ventilation system according to a second embodiment of the invention.
Figure 4 shows a front view of Figure 3.
Figure 5 shows a side view of Figure 3.
Figure 6 is a view from below of Figure 3.

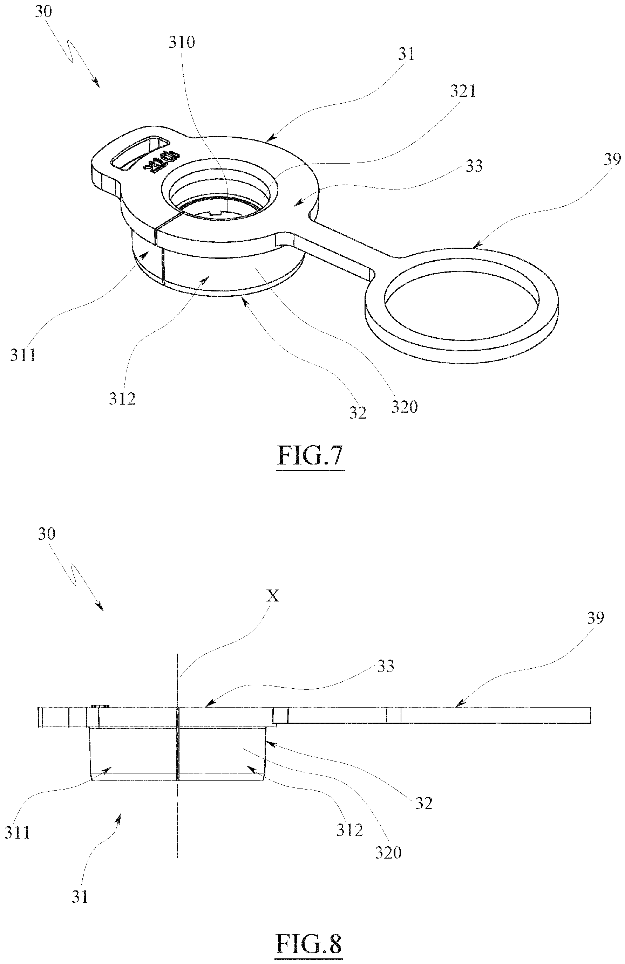

Figure 7 is an axonometric view of a connection element with which the artificial ventilation system of both the embodiments is provided.
Figure 8 shows a front view of Figure 7.

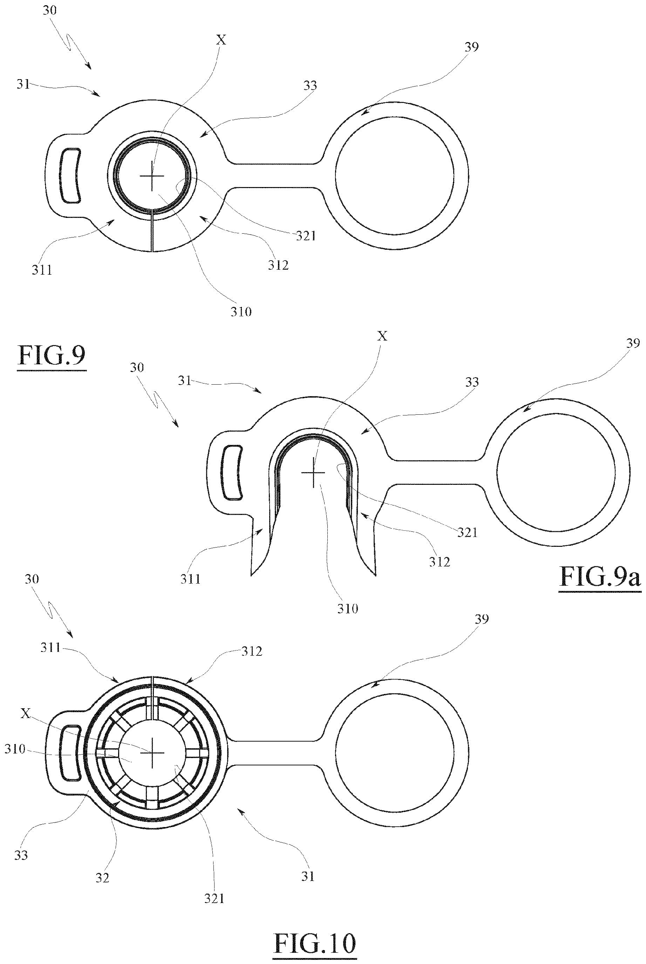

Figure 9 is a view from above of Figure 7, with the connection element in the closing configuration.
Figure 9a is a view from above of Figure 7, with the connection element in the opening configuration.
Figure 10 is a view from below of Figure 7.

### BEST MODE OF THE INVENTION

With particular reference to these figures, an artificial ventilation system is globally indicated with the number 100.

The system 100 comprises, for example, a patient interface 10', 10" adapted to contain a volume in communication with at least the mouth and nose of a patient.

The patient interface 10', 10" is provided with at least one through hole 11, the main function of which will be described better below.

For example, the patient interface 10', 10" can have a single through hole 11 (having such function) or a plurality of through holes 11 (each of which is individually - and selectively - adapted to be chosen for performing the aforesaid function for which it is intended and that will be described below).

In particular, in the embodiments illustrated in the figures, the patient interface 10', 10" is provided with a plurality of through holes 11, e.g. circular (or cylindrical) having a predetermined diameter **D***.

The patient interface 10', 10" can further comprise a means for the introduction of a breathable gas, e.g. air (or a mixture of air and oxygen or oxygen), into the volume contained by the patient interface 10', 10" and a means for the outflow of the gases exhaled by the patient towards the outside of said volume.

In particular, the introduction means comprises an inlet conduit 12 for the introduction of air (e.g. of standard dimensions for the connection to usual supply pipes of breathable gases such as air, air and oxygen or oxygen, Venturi meters or similar devices), which can be provided, at the end that leads into the volume contained by the patient interface 10', 10", with a diffuser.

Furthermore, it is not excluded that the through hole 11 or any of the through holes 11 define the aforesaid introduction means themselves.

The outflow means comprises an outlet conduit 13 for the gases exhaled by the patient when breathing (e.g. of standard dimensions for the connection to usual pipes or PEEP valves or other similar devices), which can be provided, at the end that derives from the volume contained by the patient interface 10', 10", with a diffuser.

The outlet conduit 13 and the inlet conduit 12, in some applications, can substantially coincide on the same structural element, e.g. the outlet conduit 13 in that case can be defined by a bleed valve placed on the inlet conduit 12 (also known as "vented") or can derive in a "Y" arrangement from the inlet conduit 12 itself.

The inlet conduit 12 and/or the outlet conduit 13 can have a rectilinear or curvilinear longitudinal axis and a substantially circular or any possible shaped cross section.

In the inlet conduit 12 and/or the outlet conduit 13 there may be a means to determine the pressure inside the patient interface 10',10", such as a pressure gauge.

The patient interface 10', 10" can further comprise a PEEP valve fixed (removably or permanently) to the outlet conduit 13, through which the gases exhaled by the patient during respiration exit, which therefore defines the aforesaid outflow means and guarantees that a certain positive pressure (adjustable) always persists within the volume contained by the patient interface 10', 10".

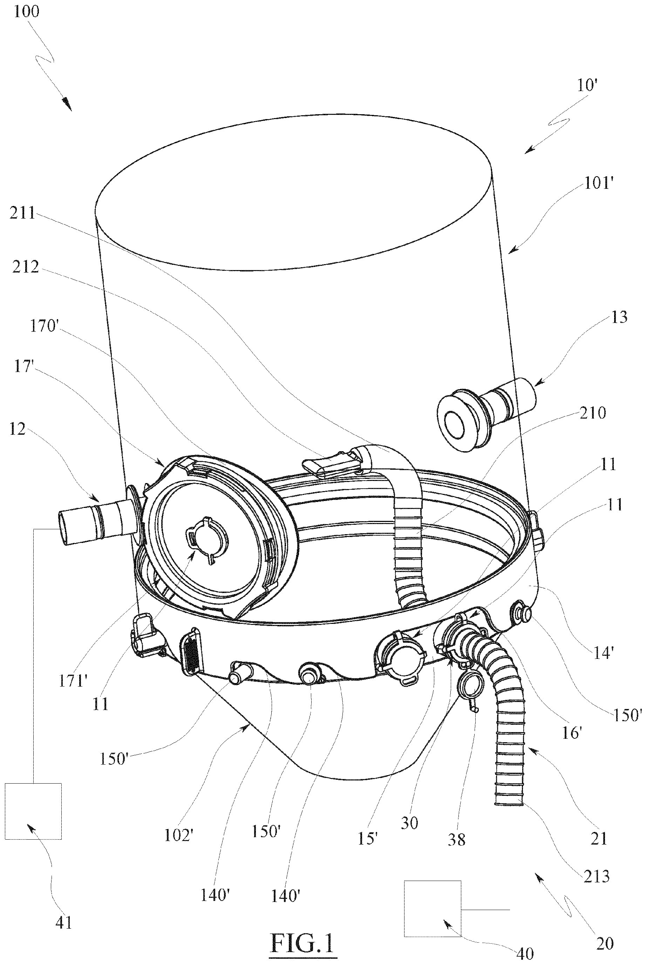

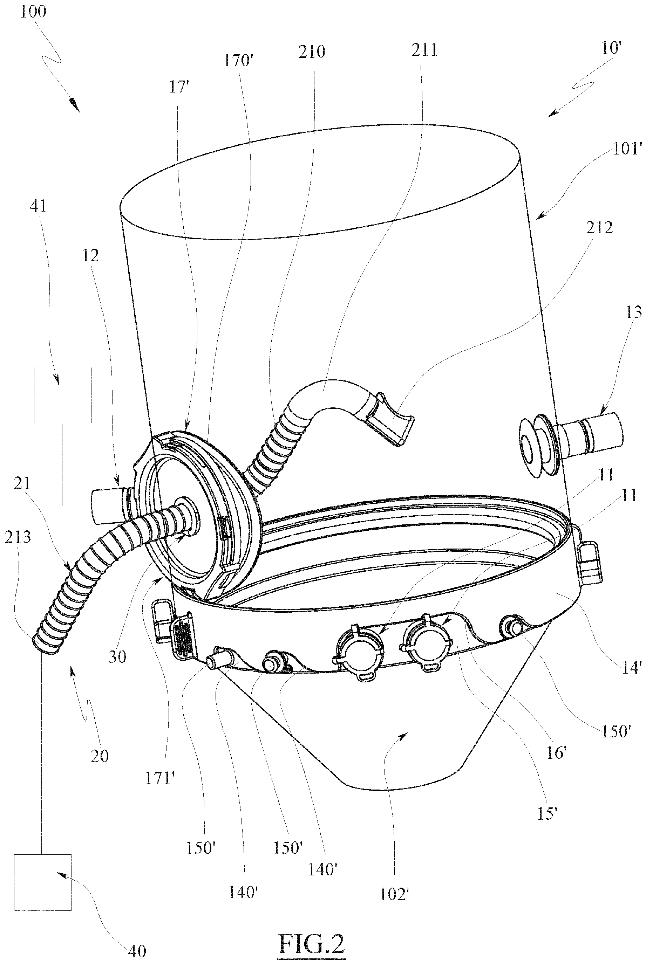

In a first embodiment of the invention shown in Figures 1 and 2, the patient interface comprises (or is constituted by) a helmet 10' in which a patient's head can be housed.

In particular, the helmet 10' comprises a container body 101', substantially cylindrical, provided with a closed upper end and an opposing open lower end.

The helmet 10' is, for example, made of an optically transparent material which, although flexible, is not dilatable.

The helmet 10' further comprises an elastically yielding collar 102', that can be sealingly coupled to the patient's neck and is adapted to close the open end of the container body 101'.

The collar 102' is advantageously made of elastically yielding material to be sealingly coupled to the patient's neck.

In practice, the collar 102' is substantially truncated cone shaped, and is provided with a first (enlarged) end and a second (tapered) end which are open.

In particular, the first end of the collar 102' has a diameter that is substantially equal to the diameter of the open lower end of the container body 101' to which said collar 102' is (removably or permanently) fixed, whereas the second end, which has smaller dimensions than the first end, has a comparable diameter or is slightly smaller than the diameter of the patient's neck.

The helmet 10' can be for example openable, however it is not excluded that, in alternative embodiments, the helmet 10' can be of the closed type.

In helmets 10' of the closed type not shown in the figures, the container body 101' is closed by the collar 102' at the open lower end thereof.

In a first variant of the helmet 10' of the closed type, the collar 102' (or the first end thereof) can be directly fixed to the container body 101' by means of fixing techniques such as heat welding or another technique. For example, in this case, the helmet 10' has no rigid structural parts such as rings or the like that would prevent it being folded away in confined spaces.

In a second variant of the helmet 10' of the closed type, between the collar 102' (i.e. the first end thereof) and the container body 101' (i.e. the open lower end thereof) a rigid ring can be interposed, which for example maintains the circular shape of the helmet 10' also when it is not being used.

In helmets 10' of the openable type shown in Figures 1 and 2, the container body 101' is connected, at the open lower end thereof, to a first rigid ring 14' by means of heat welding or another fixing technique that guarantees the hermetic and stable seal between the two. The collar 102' (i.e. the first end thereof) is connected to a second rigid ring 15', e.g. through fixing techniques that guarantee the hermetic seal between the collar 102' and the second rigid ring 15' itself.

The second rigid ring 15', which can for example be coated with soft material, can be sealingly associated, so as not to be removed, with the first rigid ring 14' (which can for example also be coated in soft material), as will appear more clearly in the following description.

The helmet 10' can further comprise an annular gasket comprised between the rigid rings when the helmet is in the closed assembly configuration.

The helmet 10' further comprises a soluble locking means 140', 150', adapted to mutually and removably lock the first rigid ring 14' and the second rigid ring 15', which are for example selected from the group from bayonet couplings, snap couplings and threaded couplings.

In particular, in the embodiment shown in Figures 1 and 2, the rigid rings are provided with locking means of the bayonet coupling type which comprise two coupling elements that are associated with the first rigid ring 14' and the second rigid ring 15', respectively. The coupling elements comprise a plurality of pins 150', for example fixed to a side surface of the second rigid ring 15' and projecting radially externally therefrom.

The coupling elements further comprise a plurality of housing seats 140', e.g. obtained in the first rigid ring 14', spaced out from each other so as to house the pins 150' of the second rigid ring 15'.

In practice, each pin 150' is adapted to be inserted removably into a housing seat 140' following a contained mutual translation-rotation between the first rigid ring 14' and the second rigid ring 15'.

In more detail, the second rigid ring 15' is adapted to be inserted substantially to measure into the first rigid ring 14' in a coaxial way.

Some embodiments of the helmet 10' can envisage that the container body 101' is provided with openings that can be closed by means of a hinge (or the like) to provide access to the patient.

The inlet conduit 12 and/or the outlet conduit 13 can be defined (fixed) at the container body 101', e.g. diametrically opposite, preferably but not necessarily having an axis directed in the radial direction with respect to a central vertical axis thereof.

However, it is not excluded that the inlet conduit 12 and/or the outlet conduit 13 can be defined (fixed) at another element constituting the helmet 10', e.g. the rigid ring (in the case of a closed helmet) or the second rigid ring 15' and/or the first rigid ring 14' (in the case of an openable type helmet).

In said first embodiment in which the patient interface is formed by the helmet 10', the helmet 10' itself, in any area thereof, comprises at least one through hole 11, which places in communication the volume inside the helmet 10' (where the patient's head is housed) with the volume outside it.

The through hole 11 may be any hole different from the inlet conduit 12 and/or from the outlet conduit 13 or be, in some circumstances, defined by at least one from among the inlet conduit 12 and the outlet conduit 13, preferably the inlet conduit 12.

In a first variant of such first embodiment, the through hole 11 is for example made in the collar 102', e.g. at the first end of the collar 102', preferably but not necessarily at the second rigid ring 15'.

In particular, the through hole 11 is made on the second rigid ring 15' connected to the collar 102', e.g. so as to cross it in the (prevalently) radial direction.

For example, the through hole 11 is (extended axially towards the outside of the helmet 10', i.e. of the second rigid ring 15' and) defined by a shank, e.g. substantially cylindrical, which has an inner diameter substantially equal to the diameter **D*** of the through hole 11 itself.

At the first rigid ring 14' connected to the container body 101', for example, a further housing seat 16' is made (defined by a circumferential split that is open at the bottom and closed at the top) adapted to surround (when the helmet 10' is in the closed assembly configuration) the area of the second rigid ring 15' affected by the through hole 11.

The helmet 10' can have a plurality of through holes 11, e.g. identical to one another.

In particular, in the embodiment shown in Figures 1 and 2, the helmet 10' comprises a pair of (identical) through holes 11, e.g. flanked in the circumferential direction (and at a contained mutual distance).

It is not excluded that the through hole 11 described above can be made in the container body 101' and/or in the first rigid ring 14' (in the openable helmet version) or in the (only) rigid ring interposed between the collar 102' and the container body 101' (in the closed type helmet version), according to the construction and use requirements.

The helmet 10' can further comprise a through opening 17', e.g. made at the container body 101', wherein the through opening 17' is adapted to place in communication the volume outside the helmet 10' with the volume contained thereby.

Said through opening 17', made for example at a front portion (facing the patient's face) of the container body 101' when the helmet 10' is worn by the patient, is sized such as to be able to be crossed by at least a hand, thus allowing the appointed personnel to operate from the outside on the head of the patient wearing the helmet 10'.

The through opening 17' can be applied, mainly, in helmets 10' of the closed type (whether they have a rigid ring or no rigid ring), as this facilitates the operations on the patient's head which could not be reached in another way, once such type of helmet is being worn.

However, it is not excluded - as in the illustrated case - that the through opening 17' is also provided in helmets 10' of the openable type, in which case allowing the equipping operations of the helmet itself and of the instruments arranged on the patient's head to be facilitated.

Said through opening 17' is preferably delimited by a rigid flange 170', e.g. with an annular shape, which for example has a substantially cylindrical shank projecting towards the outside of the container body 101'.

The rigid flange 170' is fixed to the container body 101' e.g. by means of heat welding or other appropriate fixing means.

In particular, the rigid flange 170' (i.e. the internal hole thereof) has a substantially homologous size and shape to those of the through opening 17', i.e. it is sufficiently wide to allow the appointed personnel to insert at least one hand inside the volume contained by the helmet 10', allowing sufficient manoeuvring space on the patient wearing the helmet itself.

The through opening 17' can further be sealingly closed by a plug 171', e.g. that can be removably fixed to the rigid flange 170'.

Said plug 171', in particular, comprises a disc-shaped body whose outer diameter is substantially equal to or slightly greater than the diameter of the through opening 17'.

The plug 171' comprises, for example, a cylindrical shank adapted to be inserted onto the cylindrical shank of the rigid flange 170' and to define therewith a stable soluble coupling. For example, between the cylindrical shank of the rigid flange 170' and the cylindrical shank of the plug 171' threaded or bayonet connections or snap or interference couplings or the like are defined.

In the embodiment shown in Figures 1 and 2, the cylindrical shank of the plug 171' is associated with a plurality of fixing fins equidistant from each other, which project towards the inside of the cylindrical shank and define, with respective male thread portions defined on the cylindrical shank of the rigid flange 170', the aforesaid connection.

The plug 171' can further comprise a sealing element, e.g. an annular gasket, which is adapted to be compressed (axially or radially) between the cylindrical shank of the plug 171' and the cylindrical shank of the rigid flange 170'.

In a second variant, the helmet 10' can have a through hole 11, e.g. as described above (or without a shank), at the plug 171'.

Such through hole 11 made in the plug 171' may be (in a version not illustrated) the only through hole 11 in the helmet 10' having the aforesaid main function (which will be described below) or, alternatively (as in the illustrated version), be one of the through holes 11 of the helmet 10' (from which to choose for performing the aforesaid main function). The through hole 11 made in the plug 171' is for example coaxial to the (disc-shaped body of the) plug itself and, therefore, to the through opening 17' when the plug 171' closes it.

The system 100, in said first embodiment, can further comprise, a retaining means (not shown in the figure) adapted to exercise a pulling action of the patient interface, i.e. of the helmet 10'.

In the event in which the patient interface is a helmet 10', the retaining means exercises the pulling action towards the patient's shoulders with the function of preventing the lifting of the helmet due to positive pressure inside it.

Such retaining means can be of the "underarm strap" type or the "rod" type, as known to a person skilled in the art.

Alternatively, such retaining means may comprise an internal/external cushion that can be inflated e.g. from the outside and that facilitates the contact area with the patient's neck.

Said cushion can for example be positioned below the collar 102', i.e. at the first rigid ring 14' and the second rigid ring 15'.

In this way, the structure of the helmet 10' can be made rigid, thus preventing the deformation of the collar 102' and therefore preventing the displacement effect towards the top of the helmet 10' and the variation in volume inside the container body 101'.

The internal cushion thus exercises an excellent seal on the patient's neck, avoiding the use of "underarm straps" or "rods".

Again, alternatively or additionally to the cushion, such retaining means can comprise - as known - a rigid base, as described in patent no. EP237616 by the same Applicant.

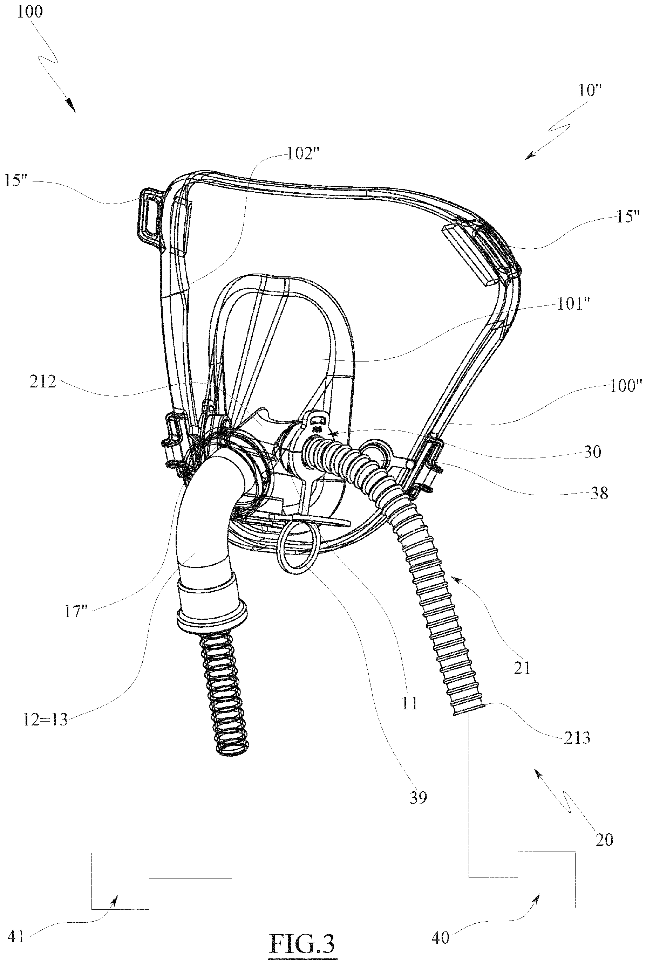

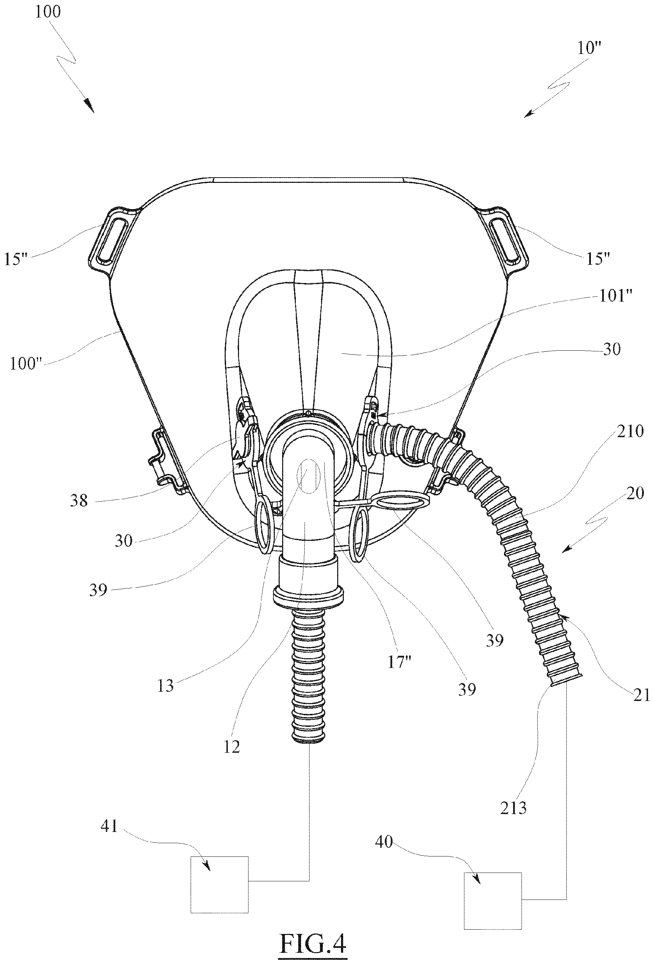

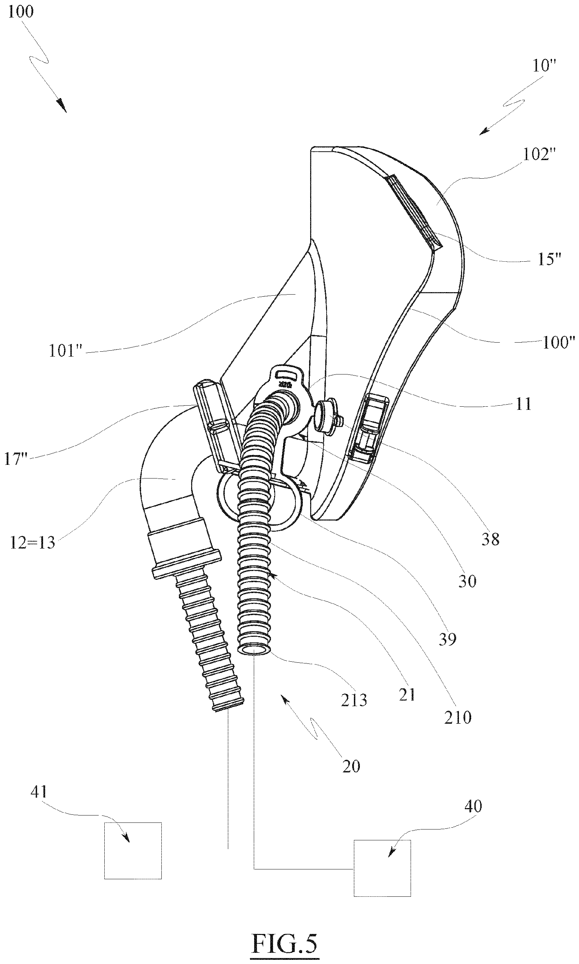

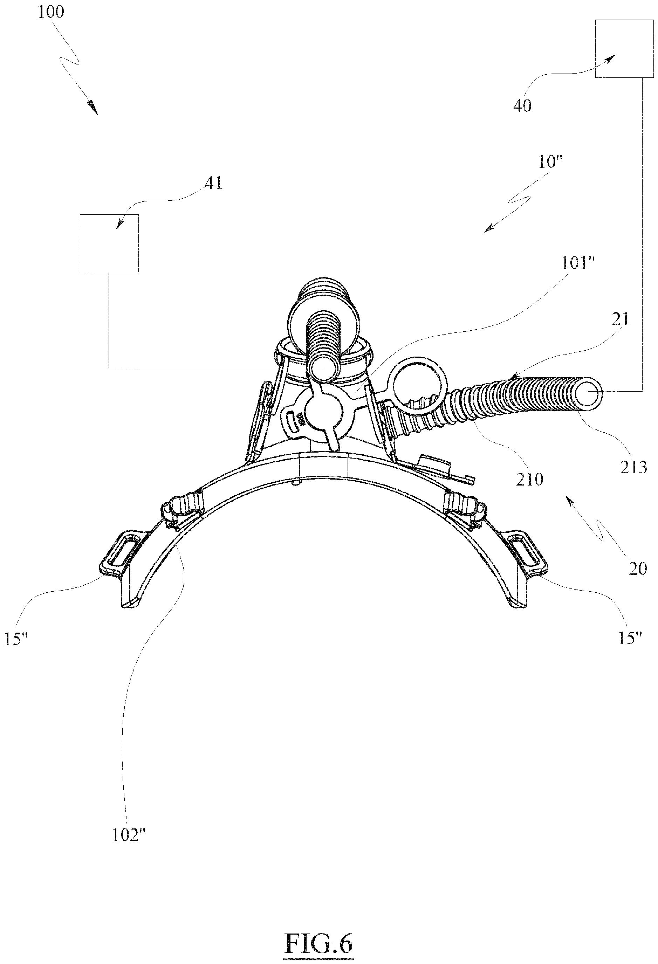

In a second embodiment of the invention shown in Figures 3-6, the patient interface comprises (or is comprised by) a mask 10", e.g. a full-face mask, i.e. that can be coupled to the patient's face, so as to inscribe the patient's mouth, nose and eyes.

It is not excluded that the mask 10 may be of the oronasal type, i.e. adapted to surround the patient's nose and mouth only.

For example, the mask 10" can be of the rigid or semi-rigid type, i.e. be shaped according to its own shape, e.g. not modifiable following the operating pressure to which the mask itself is subjected.

Furthermore, it is not excluded that the mask 10" may be, for example, made without rigid structural parts that determine overall a predefined undeformable or substantially undeformable shape thereof.

In that case, the mask 10" can be, for example, an inflatable mask, inflatable meaning that it can pass alternatively between a deflated configuration and a different inflated configuration, wherein in the inflated configuration the volume of the mask is different, preferably greater, than the volume of the mask in the deflated configuration.

The mask 10", in any case, comprises a shell 101", substantially concave, with concavity facing towards the patient's face.

The shell 101" therefore, has a concave inner side and an opposing convex outer side.

The shell 101" is, for example, made of an optically transparent material. In the event in which the mask 10" is a rigid or semi-rigid mask, the shell 101" could be made of a substantially rigid transparent polymer material, e.g. polycarbonate (PC) or another material, such as copolyester (PETG).

In the event in which the mask 10" is an "inflatable" type mask, the shell 101" could be made of a substantially flexible transparent polymer material which, although flexible, is not dilatable, e.g. as described in patent no. EP3558433 by the same Applicant.

In practice, said shell 101" projects outwards, on the opposite side with respect to the patient's face, inscribing the patient's nose and mouth (and eyes).

The shell 101" is advantageously surrounded by a perimeter edge 100", which perimeter edge 100" has a substantially trapezoidal shape (with rounded edges), e.g. an isosceles trapezium, with the smaller base arranged at the bottom during use (in the chin area) and the larger base placed at the top (in the forehead area).

In practice, the smaller base 100" of the perimeter edge is adapted to rest (although not directly) on the patient's chin (below their mouth), a larger base is adapted to rest (although not directly) on the patient's forehead (above their eyes) and the two sides that join the smaller base to the larger base are adapted to rest (although not directly) on the side of the face, i.e. around the eyes and cheeks (to the side of the eyes, nose and mouth).

Therefore, when the mask 10" rests on the patient's face, it delimits a closed volume communicating, through the patient's mouth and nose, with the respiratory system of the actual patient.

In particular, the mask 10" comprises, for example, an annular gasket 102".

In practice, the annular gasket 102" surrounds the entire perimeter of the perimeter edge 100" of the mask 10" and defines the end portion thereof adapted to come into contact with the patient's face.

The annular gasket 102" could be defined in a single body (e.g. made by overmoulding) with the shell 101" or be fixed thereto (permanently or removably) as known to a person skilled in the art.

Furthermore, the annular gasket 102" could be of the inflatable type or be made of a full or pre-inflated frame, as known in the sector.

For example, the annular gasket 102" is made of an elastomeric material (deformable in an elastic or elasto-plastic way), e.g. made of TPE or silicone or the like.

The shell 101" could have a shaped portion that projects from the front surface of the shell 101" towards the outside, about at the centre thereof, which is configured to be substantially at the nose and/or mouth of the patient when wearing the mask 10".

Such shaped portion has a substantially conical / truncated cone shape.

The inlet conduit 12 and/or the outlet conduit 13 may be defined (fixed) at the shell 101", e.g. coinciding in the same conduit and/or deriving from the same area of the shell 101" or deriving from separate / distinct areas of the shell 101", e.g. arranged symmetrically with respect to a sagittal median plane of the mask 10", preferably but not necessarily having an incident axis ideally on the side of the concavity of the shell 101".

In the example depicted, the inlet conduit 12 and/or the outlet conduit 13 are made (jointly or separately) at the shaped portion, as will be better described below, e.g. deriving (jointly or separately) therefrom (towards the outside of the shell 101").

Preferably, but not necessarily, on the shell 101" there is a through opening 17", which is for example made at the top of the shaped portion.

The through opening 17" is for example circular and, preferably, delimited by a cylindrical shank that projects towards the outside of the shell 101".

For example, the through opening 17" is made centrally on the shell 101", i.e. it is centred on the sagittal median plane of the mask 10".

At least one from among the inlet conduit 12 and the outlet conduit 13 is grafted onto the through opening 17". i.e. it is sealingly connected thereto.

Preferably but not necessarily, between the through opening 17" (i.e. the cylindrical shank thereof) and at least one from between the inlet conduit 12 and the outlet conduit 13 a rotatable connection is defined, i.e. at least one from among the inlet conduit 12 and the outlet conduit 13 is rotatably associated, about a central axis of the through opening 17", with the shell 101".

In the example depicted, at least one from among the inlet conduit 12 and the outlet conduit 13 has a curvilinear longitudinal extension, e.g. L-shaped.

For example, the inlet conduit 12 and the outlet conduit 13, in the example depicted, coincide, in fact defining a so-called "vented" conduit, i.e. provided with a bleed vale (on the extrados) from which the gases exhaled by the patient exit.

In that case, the inlet conduit 12 and the outlet conduit 13 comprise a distal end that is adapted to be connected to a supply circuit of a breathable gas (under pressure) and a proximal end grafted into the through opening 17", the bleed valve being provided in an intermediate stretch of the conduit interposed axially between the proximal end and the distal end.

However, it is not excluded that the inlet conduit 12 and the outlet conduit 13 can be integrated in a Y-shaped connector, provided with a common proximal end grafted into the through opening 17", a first (free) distal end (which in fact defines the inlet conduit 12), which is adapted to be connected to a supply circuit of a breathable gas (under pressure) and a second (free) distal end (which in fact defines the outlet conduit 13), which is adapted to be connected to a bleed valve, e.g. a PEEP valve, as described above.

In a further embodiment not shown in the figures, the inlet conduit 12 and the outlet conduit 13 can derive from distinct parts of the shell 101", e.g. of the shaped portion, e.g. from the top and/or from the conical side wall thereof.

In said second embodiment in which the patient interface is formed by the mask 10", the mask 10" itself comprises at least one through hole 11, which places in communication the volume inside the mask 10" (contained by the patient's face) with the volume outside it.

The through hole 11 is for example made at the shell 101", preferably but not necessarily in the shaped portion thereof.

The through hole 11 may be any hole different from the inlet conduit 12 and/or from the outlet conduit 13 or be, in some circumstances, defined by at least one from among the inlet conduit 12 and the outlet conduit 13, preferably the inlet conduit 12.

For example, the through hole 11 may be (extended axially towards the outside of the mask 10', and) defined by a shank, e.g. substantially cylindrical, which has an inner diameter substantially equal to the diameter **D*** of the through hole 11 itself.

In the example illustrated, the through hole 11 does not envisage such shank, i.e. it has an axial thickness equal to the thickness of the wall of the shell 101" in which it is made and has a diameter **D***, as defined above.

In one embodiment, the through hole 11 can be made in the shaped portion, e.g. in the conical side wall thereof, e.g. in the right hemisphere (thereof or) of the shell 101" or in the left hemisphere or centred on the median sagittal plane of the mask 10", e.g. in the lower area of the (conical side wall of the) shaped portion.

In particular, in the embodiment shown in Figures 3-6, the mask 10" can have a plurality of through holes 11, e.g. identical to one another.

For example, the through holes 11 of the plurality of through holes 11 have their axes incident to one another on the side of the concavity (of the shaped portion) of the shell 101".

For example, the mask 10" comprises two or three through holes 11.

In particular, a through hole 11 of the plurality of through holes 11 is made at a lower portion of the shell 101", placed in proximity to the patient's chin, e.g. centred on the median sagittal plane of the mask 10", e.g. in the lower area of the (conical side wall of the) shaped portion.

An (other) through hole 11 of the plurality of through holes 11 is made at a side portion of the shell 101", e.g. in the right hemisphere (of the conical side wall) of the shaped portion. An (other) through hole 11 of the plurality of through holes 11 is made at a (further) side portion of the shell 101", e.g. in the left hemisphere (of the conical side wall) of the shaped portion, e.g. the through holes 11 placed on the respective side portions of the shell 101" are symmetrical to one another with respect to the sagittal median plane of the mask 10". The system 100, in said second embodiment, can further comprise, a retaining means (not illustrated in the figures) adapted to exercise a pulling action of the patient interface 10', 10", i.e. of the mask 10".

In the event in which the patient interface is a mask 10", the retaining means exercises the pulling action towards the patient's face with the function of retaining the mask 10" on the patient's face, i.e. with its annular gasket 102" in contact (forced and sealingly) with the face and, therefore, allowing the establishment of positive pressure inside the volume contained between the mask 10" and the patient's face.

The retaining means is of the neck type or a head brace or another suitable system.

In particular, the retaining means is adapted to be fixed to one or more fixing means made on the mask 10", i.e. on the shell 101" thereof, preferably at the perimeter edge 100".

In the example, there is a plurality of fixing means spaced out from one another along the perimeter edge 100".

For example, each of the fixing means can be defined by a slot (or a hole), e.g. defined at a fin 15" or made at a part of the perimeter edge 100" or the like, such as a button or hook, a strap or the like.

For example, there may be four or more fixing means, as known to a person skilled in the art.

The system 100, i.e. the patient interface 10',10" in general, can further comprise an anti-suffocation valve (not shown in the figure), i.e. a two-directional valve that can place in communication the outside with the inside of the patient interface 10', 10" in case of emergency, i.e. when a drop in pressure below a threshold limit value (e.g. even greater than ambient pressure or equal to ambient pressure) is required within the volume contained by the patient interface 10', 10". For example, the anti-suffocation valve may be of the two-directional type as described in patent EP 1 797 925 in the name of the same Applicant.

However, it is not excluded that the anti-suffocation valve may be positioned on any shank (surrounding any one from among a through hole 11 or the inlet conduit 12 or the like) especially provided and placed in any desired position of the patient interface 10', 10". The system 100 further comprises a nasal cannula 20 configured for the administration of a breathable gas directly into the patient's nose.

For example, the nasal cannula 20 is configured to be supported at the patient's nose simultaneously to the patient interface 10', 10", i.e. when the same patient is wearing the patient interface 10', 10".

In particular, the nasal cannula 20 is used for the application of humidified or high flow oxygen therapy (i.e. the nasal cannula 20 is a so-called HFNC cannula).

The nasal cannula 20 comprises a hose 21, provided with a central portion 210, preferably extensible, e.g. of the corrugated hose type, a first end 211 provided with a nasal dispenser 212 and an opposing second end 213, e.g. provided with a connector (standard, not illustrated).

In detail, the central portion 210 of the nasal cannula 20 has an outer diameter **d*** (e.g. the minimum diameter, when the hose is corrugated) and an inner diameter **a*.**

The connector placed at the second end 213 (not shown in the figure) can have a larger outer diameter (or equal) with respect to the outer diameter **d*** of the central portion 210, i.e. a maximum outer diameter **dmax.**

The hose 21, preferably only the central portion 210, can be for example made of a breathable material, or at least partially breathable, with respect to a water vapour contained in the breathable gas.

Furthermore, the hose 21, preferably only the central portion 210, is adapted to emit towards the outside thereof a quantity of water vapour transported inside it, e.g. contained in the breathable gas.

The hose 21 has a length that is substantially comparable to the perimeter of a head circumference of the patient.

The nasal dispenser 212 is fixed, for example permanently or removably, to the first end 211 of the (hose 21 of) the nasal cannula 20.

The nasal dispenser 212, e.g. provided with one or two dispensing nozzles, is adapted to dispense the breathable gas directly into the patient's nostrils.

Each nozzle is configured to close more than 50% of the inner diameter of the patient's individual nostril.

Furthermore, each nozzle must be sufficiently wide so as not to compromise the patient's nasal septum.

A single nozzle can, for example, be used for neonatal therapy.

The outer diameter of each nozzle is comprised between 1.5 mm and 4.8 mm, in particular for newborns and infants the outer diameter is comprised between 1.5 mm and 1.9 mm, for paediatric patients the outer diameter is comprised between 1.9 mm and 2.7 mm and for adult patients the outer diameter is comprised between 2.7 mm and 4.8 mm, e.g. substantially equal to 4.8 mm.

The nasal cannula 20 can further comprise an anchoring unit (not shown in the figure) of the nasal dispenser 212 to the patient's face.

Said anchoring unit can be defined by an elastic annular body adapted to encircle the patient's head and to be anchored thereto.

For example, such anchoring unit can comprise a pair of laces fixed to the first end 211 of the hose 21 and branching from opposite sides with respect to the nasal dispenser 212.

Then, a respective portion of a strap, e.g. elastic, can be fixed to each of the laces, adapted to be fitted onto the patient's head for retaining the nasal dispenser 212 in proximity to the patient's nose, without engaging the actual patient's mouth or eyes.

The nasal cannula 20 is intended, during use of the system 100, to be at least partially placed inside the volume contained by the patient interface 10', 10".

In more detail, during use of the system 100, at least the nasal dispenser 212 (placed at the first end 211 of the hose 21) is arranged inside the volume contained by the patient interface 10', 10", as will be better described below.

For example, also at least one axial stretch proximal to the first end 211 (and containing it) of the (central portion 210 of the) hose 21 can be arranged inside the volume contained by the patient interface 10', 10".

Preferably, the connector of the nasal cannula 20 or at least a part thereof (with or without a further axial stretch proximal to the second end 213 and containing it) is intended, during use of the system 100, to be at least partially placed outside the volume contained by the patient interface 10', 10".

Preferably, as will be better described below, the nasal cannula 20 is adapted to be inserted, at least partially, into a through hole 11 of the patient interface 10', 10", i.e. any one of the through holes 11 (when more than one is provided).

Such, chosen, through hole 11 therefore performs the main function of housing within it the nasal cannula 20, i.e. of allowing the nasal dispenser 212 to cross the patient interface 10', 10", to reach and be installed at the nose of the patient who is wearing the same patient interface 10', 10".

The artificial ventilation system 100 further comprises a connection element 30, which is configured to connect the nasal cannula 20 to the patient interface 10', 10", guaranteeing at the same time the pneumatic seal of the system 100.

In practice, the connection element 30 is configured to allow the internal volume of the interface 10', 10" to remain, also when the nasal cannula 20 is connected thereto, substantially hermetically closed onto the (neck or face of the) patient, allowing them to be subjected to positive internal pressure suitable for the dispensing of CPAP or NIV therapy. The connection element 30 is configured to connect the nasal cannula 20 to the patient interface 10', 10", guaranteeing at the same time the pneumatic seal of the system 100. The connection element 30, in the preferred embodiment, is configured to define a gasket between the nasal cannula 20 and (the walls of) the through hole 11 into which it is inserted, i.e. to be inserted between (the outer surface of) the nasal cannula 20 and (the walls of) the through hole 11 into which it is inserted for the purpose of ensuring the pneumatic (hermetic) seal with respect to the gases.

The connection element 30 in practice, is configured to compensate and close a gap between (the outer surface of) the nasal cannula 20 and (the walls of) the through hole 11 into which it is inserted.

Furthermore, the connection element 30 is configured to axially retain (along the axis of the through hole 11) the nasal cannula 20, in fact preventing it from being extracted (when connected).

In practice, the connection element 30 defines a support of the patient interface 10',10" for the nasal cannula 20, when it is connected to the through hole 11 of the patient interface 10',10".

The connection element 30 is preferably removably connected to at least one from among the patient interface 10', 10" and the nasal cannula 20, e.g. to both.

The connection element 30 is, at least partially, inserted (removably) axially into the through hole 11.

Preferably, the connection element 30 is adapted to be inserted (removably) to measure into the through hole 11 of the patient interface 10', 10" and, at the same time, is configured to be fitted (permanently or removably) sealingly onto the nasal cannula 20.

The connection element 30 advantageously comprises a sleeve 31 that can be snugly fitted into the through hole 11 and provided with an axially through central cavity 310 with a through axis X substantially parallel (in use) to the axis of the through hole 11, e.g. coaxial therewith.

The sleeve 31 comprises a cylindrical stretch 32, provided with a cylindrical outer jacket 320 and an inner wall 321, also substantially cylindrical (coaxial to the cylindrical stretch) which delimits and defines the central cavity 310.

For example, at an axial end of the sleeve 31, i.e. of the cylindrical stretch 32 thereof, there is a flange 33, e.g. annular, which preferably has an enlarged radial direction with respect to the radial dimension (diameter) of the cylindrical stretch 32.

The flange 33 has a larger radial dimension than the diameter **D*** of the through hole 11, so as to define an abutment surface of the sleeve 31 on the perimeter edge of the through hole 11.

The flange 33, in particular, is defined at the axial end of the sleeve 31 intended to be arranged outside the volume contained by the patient interface 10', 10" (when the sleeve 31 is inserted into the through hole 11). Therefore, the flange 33 is, overall, placed outside the volume contained by the patient interface 10', 10" (when the sleeve 31 is inserted into the through hole 11).

The cylindrical stretch 32 of the sleeve 31 could be defined, at least in part, by two coaxial separate cylinders, e.g. an outer cylinder and an inner cylinder, mutually joined by means of the flange 33 (at one end) and/or radial walls (that extend longitudinally along the axis into the gap between the inner cylinder and the outer cylinder).

The inner cylinder could have a shorter axial length than the axial length of the outer cylinder which, therefore, projects axially on the opposite side with respect to the flange 33 beyond the inner cylinder.

The inner wall 321, in this case, is defined by the inner wall of the inner cylinder and the outer cylindrical jacket 320 is defined by the outer wall of the outer cylinder.

In any case, the cylindrical stretch 32 has, overall, an outer diameter **D** (which corresponds, for example, to the diameter of the outer cylindrical jacket 320) and an inner diameter **d** (that corresponds for example to the diameter of the inner wall 321).

The cylindrical stretch 32 is, preferably, made in a single body with the flange 33.

The flange 33, further, has a central hole that extends (in fact defining an end) the central cavity 310 of the sleeve 31.

Such central hole has an inner diameter greater than or equal to the inner diameter **d** of the cylindrical stretch 32.

The cylindrical stretch 32 is, preferably, sized so as to be inserted with interference into the through hole 11 (selected).

In detail, the outer diameter **D** of the cylindrical stretch 32 is greater than or equal to the (inner) diameter **D*** of the through hole 11.

The cylindrical stretch 32 is sized so as to be fitted sealingly / by interference onto the nasal cannula 20, preferably onto an axial stretch of the hose 21 (in even more detail, of the central portion 210).

In detail, the inner diameter **D** of the cylindrical stretch 32 is less than or equal to the outer diameter **D*** of the nasal cannula 20.

The inner diameter **d** of the cylindrical stretch 32 is preferably sized so as to not excessively compress (e.g. until it chokes) the nasal cannula 20, i.e. the hose 21.

For that purpose, the inner diameter **D** of the cylindrical stretch 32 is, preferably, greater than or equal to the inner diameter **D*** of the nasal cannula 20.

The connection element 30, for example at least the cylindrical stretch 32 thereof, is preferably deformable, e.g. elastically, e.g. at least in the radial direction.

In the example, the connection element 30 is made (at least partially or fully) of a deformable material, preferably elastically, such as for example an elastomer (or a rubber) or the like.

The aforesaid outer diameter **D** preferably refers to the outer diameter in the configuration not deformed / not strained in compression of the connection element 30, and the said inner diameter **d** relates to the inner diameter in the configuration not deformed / not strained in compression of the connection element 30.

The connection element 30 can be alternatively switched between an opening configuration and a closing configuration, as will be better described below.

Advantageously, the sleeve 31, as described above, is formed by the join, along at least a longitudinal meeting plane parallel to the through axis X of the central cavity 310 of the sleeve 31, of a plurality of flaps 311, 312 which, overall, define the aforesaid sleeve 31 provided with said central cavity 310, i.e. overall they define the cylindrical stretch 32 (and the flange 33) which form the sleeve 31 itself.

The meeting plane of the flaps 311, 312 is a plane configured to cut the outer jacket 320 of the cylindrical stretch 32 and to cut (or at least be tangent to) the inner wall 321 thereof, along at least a respective directrix.

Preferably, but not necessarily, the meeting plane is a radial plane comprising the through axis X of the central cavity 310.

In the example, there are two flaps 311, 312 that form the sleeve 31, each of which, for example, is such as to separate the cylindrical stretch 32 into two semi-cylinders (in the example of the same shape).

Said two flaps 311, 312 can be operatively switched between a closing configuration, in which the central cavity 310 has a (closed and) minimum cross section (and a substantially cylindrical shape), and an opening configuration, in which the central cavity 310 has an (open and) greater cross section than the minimum cross section.

In practice, the two flaps 311, 312 can be mutually separated along the meeting plane so as to make the radial opening of the central cavity 310 possible.

In a possible embodiment the two flaps 311, 312 can be two separate bodies which, when joined, compose the said sleeve 31.

Alternatively, the two flaps 311, 312 can be joined together, e.g. at a meeting area (i.e. an axial stretch or meeting plane) extending parallel to the longitudinal axis of the sleeve 31, i.e. to the through axis X of the central cavity 310.

For example, the two flaps 311, 312 can be substantially hinged to one another, e.g. about a hinge axis parallel to the longitudinal axis of the sleeve 31, i.e. to the through axis X of the central cavity 310 (and substantially arranged diametrically - opposite the meeting plane of the flaps 311, 312).

In this way, the mutual rotation of the flaps 311, 312 is configured to cause alternative switching between the aforesaid closing configuration and the aforesaid opening configuration.

For example, the two flaps 311, 312 can be hinged by means of a relevant (physical) hinge, not illustrated.

In that case, the two flaps 311, 312 can be made of two distinct bodies joined at the aforesaid meeting area, i.e. at the said hinge.

In the preferred embodiment shown in the figures, the two flaps 311, 312 are made in a single body, i.e. they (both) derive from the common meeting area.

For example, the two flaps 311, 312 can be hinged by means of an imaginary hinge axis defined by an (imaginary) bending line between the two flaps 311, 312.

In that case, the two flaps 311, 312 both derive from the same meeting area, which comprises within it the imaginary hinge axis.

The (only) meeting area (or - as mentioned - the entire sleeve 31 and, therefore, also the flaps 311, 312) is for example deformable, e.g. elastically, so as to allow the mutual (elastic) bending of the two flaps 311, 312 about said imaginary hinge axis.

In practice, each of the two flaps 311, 312 has its own proximal circumferential end, at the said meeting area, which is joined to and derives from the proximal circumferential end of the other flap and, an opposing distal circumferential end that defines and delimits the respective meeting area of the two flaps.

In general, when the flaps 311, 312 are in their closing configuration and the distal circumferential ends are joined, e.g. closing (sealingly) onto one another in contact, so as to radially close the central cavity 310.

Furthermore, when the flaps 311, 312 are in the opening configuration, the distal circumferential ends of the flaps 311, 312 are spaced out from one another by a non-null distance (i.e. they are not in mutual contact), in fact opening the said through cavity 310 in the radial direction.

For example, the distal circumferential ends of the flaps 311, 312 may be mutually distanced by at least a minimum distance **I** such as to allow the introduction in the radial direction of the nasal cannula 20 into the through cavity 310, i.e. at least equal to (or greater than) the outer diameter **d*** of the (central portion 210 of the) nasal cannula 20, preferably but not necessarily equal to (or greater than) the maximum outer diameter **dmax** of the nasal cannula 20.

The distal circumferential ends of the flaps 311, 312 can be obtained by cutting (along the meeting plane) starting from a sleeve 31 made in a single body.

When the sleeve 31 is inserted into the (selected) through hole 11, it is (exclusively) arranged in its closing configuration (and it is prevented - by the walls of the through hole 11 itself - from being able to pass into the opening configuration.

In its closing configuration, the sleeve 31 is adapted to engage to measure the through hole 11, whereas in the opening configuration it cannot be inserted into the through hole 11 itself.

The central cavity 310 of the connection element 30, i.e. of the sleeve 31, (when it is not engaged by the nasal cannula 20) can be closed, e.g. by a cover 38 fixed to the sleeve 31, e.g. to the flange 33 thereof, by means of a connection flap (flexible, e.g. elastically). Said cover 38 is adapted to sealingly close the connection element 30 (when the nasal cannula 20 is not connected inside it), from the outside.

The connection flap derives (radially) from the outer periphery of the flange 33 e.g. squared with the meeting plane.

The connection flap and the cover 38 are defined, for example, in a single body with the sleeve 31.

It is not excluded that, in alternative embodiments, the central cavity 310 of the sleeve 31 can be sealingly closed by a membrane, possibly also affected by the cut.

The connection element 30 can further be provided with a retaining ring 39 connected to the sleeve 31 e.g. to the flange 33 thereof, through a further connection flap (flexible, e.g. elastically).

The retaining ring 39 is adapted to be fitted onto a shank (where provided) with the through hole 11 for retaining at/in proximity thereto the sleeve 31 itself (when it is not inserted into the through hole 11).

The further connection flap derives (radially) from the outer periphery of the flange 33 e.g. from the meeting area (where provided) on the diametrically opposite side to the meeting plane, e.g. squared with respect to the connection flap of the cover 38.

Such further connection flap and the retaining ring 39 are defined, for example, in a single body with the sleeve 31.

In the embodiments shown in the figures, the system 100 comprises a plurality of connection elements 30 (the same number as the number of through holes 11 present on the patient interface 10',10").

It is not excluded that the system 100 comprises a single connection element 30, as described above, i.e. the same number thereof as the number of nasal cannulae 20 of the system 100).

In that case, the through holes 11 that are not affected by the connection element 30 are occluded by a further occlusion means, such as for example a (common) SNG plug which, for example, is configured - as known - to allow the connection of conduits, cables, catheters or probes to the patient.

The system 100 is further provided with at least one supply circuit of a gas breathable by the patient (placed in the volume contained by the patient interface 10',10").

In a simplified embodiment, the supply circuit could be a single supply circuit that supplies the breathable gas both to the nasal cannula 20 and to the patient interface 10',10" (simultaneously), e.g. through (the connector placed at the second end 213 of) the nasal cannula 20.

In an alternative (and advantageous) embodiment (illustrated in the figures), the system 100 comprises a first supply circuit 40 connected (removably) to the nasal cannula 20. The first supply circuit 40 is for example a high flow (HF) circuit or a humidified oxygen therapy circuit.

For example, (the connector placed at) the second end 213 of the (hose 21 of the) nasal cannula 20 can be associated (e.g. permanently or removably fixed) to a dispenser tube of the first supply circuit 40 of the breathable gas.

For example, the first supply circuit 40 of the breathable gas comprises a breathable gas supply unit provided with elements for the regulation of at least one from among the following parameters: flow, pressure, temperature, concentration and composition of the breathable gas itself.

The breathable gas is, for example, a mixture of air, oxygen and water vapour or oxygen and water vapour or another appropriate mixture, e.g. said breathable gas may even not be wet, or not have any water vapour.

The breathable gas may also comprise helium (He), or be a helium-oxygen (and vapour) mixture, which having a lower density than air reduces the respiratory work, reducing the force required to transfer the gas through the airways.

Said breathable gas may be a hot fluid at a temperature comprised between 30°C and 40°C, e.g. between 36°C and 37°C.

For example, the breathable gas can be supplied by the first supply circuit 40 with a flow comprised between 1 l/min and 80 l/min, e.g. comprised between 1 l/min and 8 l/min (low flow) or comprised between 5 l/min and 40 l/min (high flow).

The first supply circuit 40 ends with a dispensing tube, the downstream end of which (in the flowing direction of the breathable gas dispensed) is provided with an appropriate connector, e.g. a female connector (which is directly or indirectly connected to the connector of the nasal cannula 20).

The first supply circuit 40 can for example comprise an inlet branch and an outlet branch, wherein a source of breathable gas (e.g. oxygen and/or air) is associated with the inlet branch, and the outlet branch is intended to be connected to the nasal cannula 20.

For example, a humidifier/vaporiser for the humidification of the breathable gas introduced into the nasal cannula 20 is associated with the outlet branch of the nasal cannula 20.

The connector associated with the second end 213 of the (hose 21 of the) nasal cannula 20 is configured to be connected to the female connector of the outlet branch of the first supply circuit 40, so as to connect the nasal cannula 20 to the first supply circuit 40 for the supply of the breathable gas directly into the patient's nose.

The system 100 can further comprise a second supply circuit 41 of a breathable gas, e.g. with flows and concentrations that can be appropriately regulated and calibrated as a function of the therapy (CPAP or NIV) to be imparted, wherein the second supply circuit 41 is connected (removably) to the patient interface 10', 10", so as to fill the volume contained thereby with the breathable gas supplied thereby.

For example, the second supply circuit 41 can be connected to the inlet conduit 12 or to any one of the through holes 11 (e.g. not affected by the nasal cannula 20).

The second supply circuit 41 can be independent with respect to the first supply circuit 40.

However, it is not excluded that the two supply circuits 40, 41 may be interconnected by an appropriate Y-shaped connector, provided with an inlet branch and two outlet branches, wherein a source of breathable gas (e.g. oxygen and/or air) is associated with the inlet branch, one of the two outlet branches is intended to be connected to the nasal cannula 20 and the other outlet branch to the patient interface 10',10" (i.e. to one from among the inlet conduit 12 and a through hole 11).

The second supply circuit 41 is for example suitable to supply a breathable (dry) gas, such as for example air or an air-oxygen mixture (as known for CPAP and/or NIV therapy), at a greater pressure than atmospheric pressure (within the volume contained by the patient interface 10',10").

In light of the above, the operation of the system 100 is substantially as follows.

The nasal cannula 20 is connected, passing through any one of the through holes 11, to the patient interface 10',10" through the connection element 30, so that the nasal dispenser 212 is within the volume of the patient interface 10',10" intended to be closed onto the patient's ventilation system.

In practice, the connection element 30 sealingly embraces an axial stretch of the nasal cannula 20, preferably of (the central portion 210 and/or of the first end 211 of) the hose 21 and, then, is sealingly inserted axially into the through hole 11 (selected), where it (axially) retains the nasal cannula 20.

In this way, a therapy obtained from the beneficial combination of (CPAP or NIV) therapy imparted through the patient interface 10',10" and the high flow ventilation or humidified oxygen therapy imparted to the patient through the nasal cannula 20 can be effectively administered to the airways of the patient arranged within the volume contained by the patient interface 10',10".

To proceed with the installation of the system 100, the following method can for example be followed.

The nasal cannula 20 can first be associated with the patient, e.g. by encircling the head with the anchoring unit thereof and inserting or moving the nasal dispenser 212 towards the actual patient's nostrils.

Once the nasal dispenser 212 has been fitted, the appointed personnel can position the patient interface 10', 10" (or a portion thereof, such as the collar 102') in proximity to the patient's head, make the second end 213 of the nasal cannula 20 pass from the inside to the outside through a through hole 11, thus inserting the nasal cannula 20 into the through hole 11 itself.

Subsequently, it is possible to radially fit the connection element 30 onto the nasal cannula 20, first bringing it into the opening configuration and, then, closing it onto a desired axial stretch (placed outside the patient interface 10', 10") of the nasal cannula 20 (i.e. of the hose 21).

In particular, it is possible in advance to bring the flaps 311, 312 of the sleeve 31 of the connection element 30 into the opening configuration, so as to allow the inlet of the nasal cannula 20 into the central cavity 310 of the sleeve 31 in the radial direction, without having to axially insert the nasal cannula 20 into said central cavity 310.

Then, said flaps 311, 312 of the sleeve 31 can be brought back into the closing configuration, so that the sleeve 31 of the connection element 30 is fitted sealingly onto the nasal cannula 20.

Subsequently, with an axial sliding of the connection element 30 towards the patient interface 10', 10" and, therefore, of the nasal cannula 20 associated therewith, the connection element 30 is axially inserted into the through hole 11, where it sealingly closes the gap between the nasal cannula 20 and the through hole 11 itself, thus guaranteeing the pneumatic seal of the system 100 when the patient interface 10', 10" is then definitively installed on the patient.

The installation of the system 100 can be completed by connecting the (first and/or second) supply circuit 40, 41 to the nasal cannula 20 and/or to the patient interface 10'10". The invention is defined by the appended claims. Subject-matter referred as embodiments, disclosures and/or inventions which are not claimed are not part of the invention.

## Claims

1. An artificial ventilation system (100) comprising:
- a patient interface (10', 10") adapted to contain a volume in communication with at least the mouth and nose of a patient, wherein the patient interface (10', 10") is provided with a through hole (11);
- a nasal cannula (20) connected to the through hole (11) of the patient interface (10', 10"); and
- a connection element (30) snugly fitted into the through hole (11) and sealingly fitted onto the nasal cannula (20);
**characterized in that** the connection element (30) is elastically deformable.

2. The system (100) according to claim 1, wherein the patient interface comprises a helmet (10') provided with a container body (101') in which the patient's head can be housed and a collar (102') that can be sealingly coupled to the patient's neck.

3. The system (100) according to claim 1, wherein the patient interface comprises a full-face mask (10") provided with a perimeter edge (100") that can be coupled to the patient's face and configured to inscribe the patient's mouth, nose and eyes.

4. The system (100) according to claim 1, wherein the connection element (30) comprises a sleeve (31) that can be snugly fitted into the through hole (11) and provided with a central cavity (310).

5. The system (100) according to the preceding claim, wherein the sleeve (31) comprises two flaps (311, 312) that surround and define the central cavity (310), wherein the two flaps (311, 312) are operatively switchable between a closing configuration, in which the central cavity (310) has a minimum section, and an opening configuration, in which the central cavity (310) has a larger section than the minimum section.

6. The system (100) according to the preceding claim, wherein the two flaps (311,312) are made of a single body and can each have a proximal end that derives from the proximal end of the other flap (312,311) and an opposing distal end separated from the distal end of the other flap (312,311).

7. The system (100) according to claim 2, wherein the patient interface (10') comprises a through opening (17'), adapted to place in communication the outside of the helmet (10') with the volume enclosed thereby, wherein the through opening (17') is sized such as to be able to be crossed by a hand and closed sealingly by a plug (171'), the said through hole (11) being made in the plug (171').

8. The system (100) according to claim 1, wherein the patient interface (10', 10") comprises a means (12) for the introduction of a breathable gas into the volume enclosed by the patient interface (10', 10") itself.

9. The system (100) according to claim 1, wherein the patient interface (10', 10") comprises a means (13) for the outflow of the gases exhaled by the patient towards the outside of the volume contained by the patient interface (10', 10").

10. The system (100) according to claim 1, wherein the nasal cannula (20) is provided with a hose (21) to a first end (211) of which a nasal dispenser (212) is fixed for the administration of a breathable gas directly into the patient's nose.

11. The system (100) according to the preceding claim, wherein at least the nasal dispenser (212) of the nasal cannula (20) is arranged within the volume enclosed by the patient interface (10', 10").

12. The system (100) according to claim 10, wherein the hose (21) of the nasal cannula (20) is at least partially breathable for a water vapour contained in the breathable gas.

## Patentansprüche

1. Künstliches Beatmungssystem (100), umfassend:
- eine Patientenschnittstelle (10', 10"), die angepasst ist, um ein Volumen in Kommunikation mit mindestens dem Mund und der Nase eines Patienten zu enthalten, wobei die Patientenschnittstelle (10', 10") mit einem Durchgangsloch (11) versehen ist;
- eine Nasenkanüle (20), die mit dem Durchgangsloch (11) der Patientenschnittstelle (10', 10") verbunden ist; und
- ein Verbindungselement (30), das engsitzend in das Durchgangsloch (11) eingesetzt und dicht auf die Nasenkanüle (20) aufgesetzt ist;
**dadurch gekennzeichnet, dass** das Verbindungselement (30) elastisch verformbar ist.

2. System (100) nach Anspruch 1, wobei die Patientenschnittstelle einen Helm (10') umfasst, der mit einem Behälterkörper (101'), in dem der Kopf des Patienten untergebracht werden kann, und einem Bund (102'), der abdichtend mit dem Hals des Patienten verbunden werden kann, versehen ist.

3. System (100) nach Anspruch 1, wobei die Patientenschnittstelle eine Vollgesichtsmaske (10") umfasst, die mit einem Umfangsrand (100") versehen ist, der mit dem Gesicht des Patienten gekoppelt werden kann und konfiguriert ist, um den Mund, die Nase und die Augen des Patienten einzubeschreiben.

4. System (100) nach Anspruch 1, wobei das Verbindungselement (30) eine Hülse (31) umfasst, die engsitzend in das Durchgangsloch (11) eingesetzt werden kann und mit einem mittleren Hohlraum (310) versehen ist.

5. System (100) nach dem vorherigen Anspruch, wobei die Hülse (31) zwei Klappen (311, 312) umfasst, die den mittleren Hohlraum (310) umgeben und definieren, wobei die zwei Klappen (311, 312) funktionsfähig zwischen einer Schließkonfiguration, in der der mittlere Hohlraum (310) einen minimalen Querschnitt aufweist, und einer Öffnungskonfiguration, in der der mittlere Hohlraum (310) einen größeren Querschnitt als den minimalen Querschnitt aufweist, umschaltbar sind.

6. System (100) nach dem vorherigen Anspruch, wobei die zwei Klappen (311, 312) aus einem einzigen Körper gefertigt sind und jeweils ein proximales Ende, das von dem proximalen Ende der anderen Klappe (312, 311) abgeleitet ist, und ein gegenüberliegendes distales Ende, das von dem distalen Ende der anderen Klappe (312, 311) getrennt ist, aufweisen können.

7. System (100) nach Anspruch 2, wobei die Patientenschnittstelle (10') eine Durchgangsöffnung (17') umfasst, die angepasst ist, um die Außenseite des Helms (10') mit dem davon umschlossenen Volumen in Verbindung zu bringen, wobei die Durchgangsöffnung (17') so bemessen ist, um in der Lage zu sein, von einer Hand durchquert und durch einen Stopfen (171') dicht verschlossen zu werden, wobei das Durchgangsloch (11) in dem Stopfen (171') gefertigt ist.

8. System (100) nach Anspruch 1, wobei die Patientenschnittstelle (10', 10") eine Einrichtung (12) für die Einleitung eines atembaren Gases in das von der Patientenschnittstelle (10', 10") an sich umschlossene Volumen umfasst.

9. System (100) nach Anspruch 1, wobei die Patientenschnittstelle (10', 10") eine Einrichtung (13) zum Ausströmen der vom Patienten ausgeatmeten Gase in Richtung der Außenseite des von der Patientenschnittstelle (10', 10") enthaltenen Volumens umfasst.

10. System (100) nach Anspruch 1, wobei die Nasenkanüle (20) mit einem Schlauch (21) versehen ist, an dessen erstem Ende (211) ein Nasenspender (212) zur Verabreichung eines atembaren Gases direkt in die Nase des Patienten befestigt ist.

11. System (100) nach dem vorherigen Anspruch, wobei mindestens der Nasenspender (212) der Nasenkanüle (20) innerhalb des von der Patientenschnittstelle (10', 10") umschlossenen Volumens angeordnet ist.

12. System (100) nach Anspruch 10, wobei der Schlauch (21) der Nasenkanüle (20) mindestens teilweise für einen im Atemgas enthaltenen Wasserdampf atembar ist.

## Revendications

1. Système de ventilation artificielle (100) comprenant :
- une interface patient (10', 10") adaptée pour contenir un volume en communication avec au moins la bouche et le nez d'un patient, dans laquelle l'interface patient (10', 10") est pourvue d'un trou débouchant (11) ;
- une canule nasale (20) reliée au trou débouchant (11) de l'interface patient (10', 10"); et
- un élément de liaison (30) ajusté dans le trou débouchant (11) et monté de manière étanche sur la canule nasale (20) ;
**caractérisé en ce que** l'élément de liaison (30) est élastiquement déformable.

2. Système (100) selon la revendication 1, dans lequel l'interface patient comprend un casque (10') fourni avec un corps de récipient (101') dans lequel peut être logé la tête du patient et un collier (102') pouvant être relié de manière étanche au cou du patient.

3. Système (100) selon la revendication 1, dans lequel l'interface patient comprend un masque complet (10") muni d'un bord périphérique (100") pouvant être relié au visage du patient et configuré pour couvrir la bouche, le nez et les yeux du patient.

4. Système (100) selon la revendication 1, dans lequel l'élément de liaison (30) comprend un manchon (31) pouvant être ajusté dans le trou débouchant (11) et muni d'une cavité centrale (310).

5. Système (100) selon la revendication précédente, dans lequel le manchon (31) comprend deux rabats (311, 312) qui entourent et définissent la cavité centrale (310), dans lequel les deux rabats (311, 312) peuvent être commutés de manière opérationnelle entre une configuration de fermeture, dans laquelle la cavité centrale (310) a une section minimale, et une configuration d'ouverture, dans laquelle la cavité centrale (310) a une section plus grande que la section minimale.

6. Système (100) selon la revendication précédente, dans lequel les deux rabats (311, 312) sont constitués d'un seul corps et peuvent chacun avoir une extrémité proximale dérivée de l'extrémité proximale de l'autre volet (312, 311) et une extrémité distale opposée séparée de l'extrémité distale de l'autre rabat (312, 311).

7. Système (100) selon la revendication 2, dans lequel l'interface patient (10') comprend une ouverture débouchante (17'), adaptée pour mettre en communication l'extérieur du casque (10') avec le volume enfermé par celui-ci, dans lequel l'ouverture débouchante (17') est dimensionnée de manière à pouvoir être traversée par une main et fermée de manière étanche par un bouchon (171'), ledit trou débouchant (11) étant réalisé dans le bouchon (171').

8. Système (100) selon la revendication 1, dans lequel l'interface patient (10', 10") comprend un moyen (12) permettant l'introduction d'un gaz respirable dans le volume enfermé par l'interface patient (10', 10") elle-même.

9. Système (100) selon la revendication 1, dans lequel l'interface patient (10', 10") comprend un moyen (13) d'écoulement des gaz exhalés par le patient vers l'extérieur du volume contenu par l'interface patient (10', 10").

10. Système (100) selon la revendication 1, dans lequel la canule nasale (20) est munie d'un tuyau (21) à une première extrémité (211) duquel est fixé un distributeur nasal (212) destiné à administrer un gaz respirable directement dans le nez du patient.

11. Système (100) selon la revendication précédente, dans lequel au moins le distributeur nasal (212) de la canule nasale (20) est disposé dans le volume circonscrit par l'interface patient (10', 10")

12. Système (100) selon la revendication 10, dans lequel le tuyau (21) de la canule nasale (20) est au moins partiellement respirable pour une vapeur d'eau contenue dans le gaz respirable.
